# EUROPÄISCHE PATENTANMELDUNG

(11) **EP 1 060 708 A2**
(43) Veröffentlichungstag der Anmeldung: **20.12.2000**
(21) Anmeldenummer: 00890174.6
(22) Anmeldetag: 30.05.2000
(51) Int. Cl.: A61B 5/15

(54) **Programm für die Steuerung eines Gerätes zur Optimierung der Venenfüllung bei Blutabnahmen**

(30) Priorität: 17.06.1999 AT 41799 U
(71) Anmelder: Jauk, Franz, Dr., 1120 Wien (AT)
(72) Erfinder: Jauk, Franz, Dr., 1120 Wien (AT)
(74) Vertreter: Rippel, Andreas, Dipl.-Ing.

(57) **Zusammenfassung**

Ein Programm für die Steuerung eines Gerätes zur Optimierung der Venenfüllung bei Blutabnahmen ist für ein Gerät mit einer aufblasbaren Manschette und einem Manometer bestimmt.

Dieses Programm zeichnet sich dadurch aus, daß die Manschette bis zu einem wählbaren Manschettendruck aufgepumpt wird, daß der Druck in der Manschette abgesenkt wird und der Blutdruck festgestellt wird, worauf der Druck in der Manschette um einen vorgewählten Wert weiter abgesenkt werden kann.

Durch dieses Programm können bei Einstellung des wählbaren Manschettendruckes automatisch die optimalen Werte für die Blutabnahme erreicht werden.

## Beschreibung

Die Erfindung bezieht sich auf ein Programm für die Steuerung eines Gerätes zur Optimierung der Venenfüllung bei Blutabnahmen, wobei das Gerät eine aufblasbare Manschette und ein Manometer aufweist.

Die Blutabnahme erfolgt durch Einführen einer Venüle in eine Vene der Ellenbeuge, wobei die Geschwindigkeit, mit der das Blut abfließt, durch Abbinden mit einer Manschette geregelt wird. Die Geschwindigkeit ist demnach vom Blutdruck abhängig und es bleibt dem Geschick des Arztes überlassen, durch mehr oder weniger Abbinden den jeweiligen Blutdruck und damit die Geschwindigkeit des Abfließens zu bestimmen. Der Blutdruck wird dabei zwangsläufig unter dem höchsten, nämlich dem systolischen Druck liegen.

Die Erfindung hat es sich zum Ziel gesetzt, ein Programm für die Steuerung eines Gerätes zur Optimierung der Venenfüllung bei Blutabnahmen zu schaffen, das eine automatische Einstellung der richtigen Druckwerte ermöglicht. Dieses Programm soll bei einem Gerät anwendbar sein, das eine aufblasbare Manschette und ein Manometer aufweist.

Erfindungsgemäß zeichnet sich dieses Programm dadurch aus, daß die Manschette bis zu einem wählbaren Manschettendruck aufgepumpt wird, daß der Druck in der Manschette abgesenkt wird und der Blutdruck festgestellt wird, worauf der Druck in der Manschette um einen vorgewählten Wert weiter abgesenkt werden kann.

Wird ein solches Programm bei einem Gerät der genannten Art verwendet, werden bei Einstellung des wählbaren Manschettendruckes automatisch die optimalen Werte für die Blutabnahme erreicht.

Zweckmäßig liegt der wählbare Manschettendruck in einem Bereich bis 300 mm Hg und eine Absenkung des Druckes in der Manschette kann wählbar um 1 bis 100% oder um 1 bis 200 mm Hg erfolgen.

Um ein schnelles Arbeiten mit einem nach dem erfindungsgemäßen Verfahren arbeitenden Gerät zu ermöglichen, wird vorgeschlagen, die Manschette mit einem Schnellverschluß, z. B. einem Klettverschluß auszustatten.

## Patentansprüche

1. Programm für die Steuerung eines Gerätes zur Optimierung der Venenfüllung bei Blutabnahmen, wobei das Gerät eine aufblasbare Manschette und ein Manometer aufweist, **dadurch gekennzeichnet,** daß die Manschette bis zu einem wählbaren Manschettendruck aufgepumpt wird, daß der Druck in der Manschette abgesenkt wird und der Blutdruck festgestellt wird, worauf der Druck in der Manschette um einen vorgewählten Wert weiter abgesenkt werden kann.

2. Programm nach Anspruch 1, **dadurch gekennzeichnet**, daß der wählbare Manschettendruck in einem Bereich bis 300 mm Hg liegt und eine Absenkung des Druckes in der Manschette wählbar von um 1 - 100% oder um 1 bis 200 mm Hg erfolgen kann.

3. Gerät für ein Programm nach Anspruch 1 oder 2, **dadurch gekennzeichnet,** daß die Manschette mit einem Schnellverschluß, z. B. einem Klettverschluß ausgestattet ist.
